# EUROPÄISCHE PATENTSCHRIFT

(11) **EP 1 188 827 B1**
(45) Veröffentlichungstag und Bekanntmachung des Hinweises auf die Patenterteilung: **16.07.2008**
(21) Anmeldenummer: 01119105.3
(22) Anmeldetag: 08.08.2001
(51) Int. Cl.: C12N 15/07, C12N 15/08

(54) **Verfahren zur Fusion von dendritischen Zellen mit Tumorzellen und Medien für solche Verfahren**
Processes for the fusion of dendritic cells with tumor cells and media for such processes
Procédés pour la fusion de cellules dendritiques avec des cellules tumorales et milieux pour de tels procédés

(30) Priorität: 14.09.2000 DE 10047272
(43) Veröffentlichungstag der Anmeldung: 20.03.2002
(73) Patentinhaber: EPPENDORF AG, 22339 Hamburg (DE)
(72) Erfinder: Zimmermann, Ulrich, Prof. Dr., 97295 Waldbrunn (DE)
(74) Vertreter: Emmel, Thomas

(56) Entgegenhaltungen:
- WO-A-86/02377
- BERTSCHE U ET AL: "NUCLEAR MEMBRANE FUSION IN ELECTROFUSED MAMMALIAN CELLS" BIOCHIMICA ET BIOPHYSICA ACTA, Bd. 939, Nr. 3, 1988, Seiten 509-522, XP001018623 ISSN: 0006-3002
- SCOTT-TAYLOR T H ET AL: "Human tumour and dendritic cell hybrids generated by electrofusion: Potential for cancer vaccines." BIOCHIMICA ET BIOPHYSICA ACTA, Bd. 1500, Nr. 3, 17. März 2000 (2000-03-17), Seiten 265-279, XP001019114 ISSN: 0006-3002
- KUGLER A ET AL: "Regression of human metastatic renal cell carcinoma after vaccination with tumor cell-dendritic cell hybrids" NATURE MEDICINE, NATURE PUBLISHING, CO, US, Bd. 6, Nr. 3, März 2000 (2000-03), Seiten 332-336, XP002167052 ISSN: 1078-8956

## Beschreibung

Die Erfindung betrifft ein Verfahren nach dem Oberbegriff des Anspruches 1.

Gattungsgemäße Verfahren dienen zur Herstellung von fusionierten Zellen (Fusionsprodukten) aus dendritischen Zellen und insbesondere Tumorzellen. Dendritische Zellen sind immunoaktive Zellen, die auf ihrer Oberfläche Antigene präsentieren können und in Abhängigkeit von den präsentierten Antigenen entsprechende immunostimulierende Eigenschaften aufweisen.

Fusionsprodukte aus dendritischen Zellen und Tumorzellen können zum Einsatz kommen bei der Therapie von Tumoren. Es wird in diesem Zusammenhang auf die Veröffentlichung von Scott-Taylor H et. al. in Biophysica acta Bd. 1500 Nr 3, Seite 265-279 (2000) Kugler et al. in Nature Medicine, Vol. 6, No. 3, Seite 332 bis 336 (2000) verwiesen, die den Einsatz von mit Tumorzellen fusionierten dendritischen Zellen bei der Behandlung von metastasierenden Nierenkarzinoma beim Menschen beschreibt.

Bei dem bekannten Verfahren werden einem Patienten Tumorzellen entnommen und mit dendritischen Zellen mittels Elektrofusion miteinander verschmolzen.

Die dendritischen Zellen können aus dem Patienten selbst, aber auch aus anderen Menschen oder Tieren stammen.

Die dendritischen Zellen aber auch die Tumorzellen müssen zuvor bestrahlt werden, um z.B. Viren abzutöten.

Bei der Elektrofusion handelt es sich um ein bekanntes in aller Regel mehrschrittiges Verfahren. Eine umfassende Beschreibung kann z.B. dem Buch von U. Zimmermann & G. Neil "Electromanipulation of Cells" CRC-Pub., Bocaration Florida, 1996 entnommen werden.

In einem ersten Schritt (Pre-Alignment) werden die Zellen mittels Dielektrophorese miteinander in Kontakt gebracht. In einem zweiten Schritt (Puls) wird dann ein Puls gegeben, der den Durchbruch der Zellmembranen zur Folge hat, worauf dann die Zellmembranen und damit die Zellen fusionieren können. Um die Fusionspartner zu stabilisieren wird, werden in einem dritten Schritt (Post-Alignment) die aufgebrochenen Zellen solange mittels Dielektrophorese miteinander in Kontakt gehalten, bis die Fusion abgeschlossen ist.

Die gebildeten Fusionsprodukte (aus Tumorzellen und dendritischen Zellen) werden dem Patienten nach Bestrahlung injiziert und entfalten im Körper eine immunstimulierende Wirkung.

Nachteilig an dem beschriebenen Verfahren ist, daß die zur Herstellung der Fusionsprodukte gewählten Bedingungen keine optimalen Ergebnisse liefern.

Aufgabe der Erfindung ist es, demgegenüber ein Verfahren zur Elektrofusion von dendritischen Zellen mit erkrankten Zellen, insbesondere Tumorzellen zu schaffen, das eine höhere Ausbeute an Fusionsprodukten ermöglicht. Eine weitere Aufgabe der Erfindung ist, Medien bereitzustellen, die in dem Verfahren zur Anwendung kommen können.

Gelöst wird diese Aufgabe mit einem Verfahren, das die kennzeichnenden Merkmale des Anspruches 1 aufweist.

Bei dem erfindungsgemäßen Verfahren ist wie im Stand der Technik vorgesehen, daß die dendritischen Zellen mit den Zellen aus erkranktem Gewebe, insbesondere Tumorgewebe vermischt werden.

Vorzugsweise ist das Mischungsverhältnis bezogen auf die Zellzahl 1 zu 1. Besonders bevorzugt wird je Linie eine Zellzahl von 2 x 10⁶ Zellen (also insgesamt 4 x 10⁶ Zellen im Zellgemisch) eingesetzt.

Das Zellgemisch wird dann ggf. mit einem insbesondere isoosmolaren Waschmedium gewaschen, zentrifugiert und dann in einem Elektrofusionsmedium resuspendiert. Die resuspendierte Zellmischung wird dann z.B. in eine Elektrodenkammer eingebracht und dort elektrischen Bedingungen ausgesetzt, die zu einer Fusion der Zellen untereinander führen.

Es ist nun vorgesehen, daß das Elektrofusionsmedium eine Lösung mit folgenden Inhaltsstoffen in pyrogenfreiem, sterilem destilliertem Wasser ist:
- 20 mM/l- 150 mM/l: Zucker,
- 0,05mM/l - 1mM/l: Magnesiumsalz und
- 0,01mM/l - 1mM/l: Calciumsalz.

Im Hinblick auf die medizinische Anwendung wird bevorzugt das Elektrofusionsmedium durch Mischen von Infusionslösungen entsprechender Inhalte hergestellt.

Vorzugsweise wird als Zucker Sorbit in einer Konzentration von 75mM/l in einer Infusionslösung eingebracht, die unter dem Namen "Sorbitol-Infusionslösung 40" bei der Firma Serum-Werk Bernburg AG erhältlich ist. Anstelle dessen können aber auch andere Lösungen eingesetzt werden, die auch andere Zucker, z.B. Glukose enthalten können.

Das Magnesiumsalz wird vorzugsweise als Magnesium-L-Hydrogenglukonat-Lösung vorgesehen, die z.B. unter dem Namen "Magnesium Verla" bei der Firma Verla-Pharm GmbH erhältlich ist. Bevorzugt wird eine Konzentration von 0,5mM/l Magnesiumsalz vorgesehen. Denkbar ist natürlich auch der Einsatz von anderen Lösungen, die andere Magnesiumsalze, z.B. Magnesiumchlorid enthalten.

Das Calciumsalz wird vorzugsweise als einer Calcium-Glukonat-Disaccharat Lösung in einer bevorzugten Konzentration von 0,1 mM/l eingebracht. Eine geeignete Infusionslösung ist unter "Calcium Braun 10%" bei der Firma Braun, Melsungen erhältlich. Auch hier können andere Lösungen mit anderen Calciumsalzen in anderen Konzentrationen vorgesehen werden.

Wählt man die oben als optimal angegebenen Bedingungen, so erhält man eine Elektrofusionsmedium mit einer Leitfähigkeit von ca. 85 µS, einem pH-Wert von ca. 6,6 und einer Osmolarität von ca. 70 mOsm.

Die Osmolarität des Elektrofusionsmediums läßt sich im Rahmen der Erfindung über den Zuckergehalt in Abhängigkeit von den zu fusionierenden Zellen in einem Bereich von ca. 20 mOsm - 150 mOsm varieren.

Eine weitere Ausgestaltung der Erfindung betrifft das während des Verfahrens eingesetzte Waschmedium, das isoosmolare Eigenschaften besitzt und vorzugsweise eine Lösung mit folgenden Inhaltsstoffen in pyrogenfreiem, sterilem destilliertem Wasser ist:
- 270 mM/l- 310 mM/l: Zucker,
- 0,05mM/l- 1mM/l: Magnesiumsalz und
- 0,01mM/l- 1mM/l: Calciumsalz.

Im Hinblick auf die medizinische Anwendung wird auch das Waschmedium bevorzugt durch Mischen der z.B. oben angesprochenen Infusionslösungen hergestellt. Es können aber auch hier andere Lösungen mit anderen Zusammentsetzungen gewählt werden.

Wählt man eine Zuckerkonzentration von 290 mM/l so erhält man ein Waschmedium mit einer Leitfähigkeit von ca. 75µS, einem pH-Wert von ca. 6,3 und einer Osmolarität von ca 280 mOsm.

Die Kombination der angegebenen Waschlösung mit der erfindungsgemäß eingesetzten Elektrofusionslösung hat sich als besonders vorteilhaft herausgestellt.

In beiden Medien kann als Zucker anteilig Trehalose vorgesehen werden, oder der Zucker anteilig durch Polyethylenglykol ersetzt werden.

Eine weitere Verbesserung der Fusionsergebnisse läßt sich erzielen, wenn bei der Elektrofusion spezielle elektrische Bedingungen eingestellt werden.

Üblicherweise wird wie oben angesprochen eine 3-stufige elektrische Behandlung durchgeführt. In einem ersten Schritt lagern sich die Zellen aneinander an (Pre-Alignment). In einem zweiten Schritt werden die Zellmembranen aufgebrochen (Puls). In einem dritten Schritt werden die aufgebrochenen Zellen aneinander gehalten bis zur Stabilisierung der Fusion (Post-Alignment). Die Elektrofusion kann dabei in einer üblichen Elektrodenkammer erfolgen. Eine vorteilhafte Ausgestaltung der Erfindung sieht vor, daß Mikro- oder Nanoelektrodenstrukturen zum Einsatz kommen, die mittels Halbleitertechnologie aufgedampte Elektroden aufweisen.

Der Einsatz von Mikro- oder Nanostrukturen erlaubt eine Automatisierung des Verfahrens. Außerdem lassen sich hierin besonders elegant gezielte pärchenweise Fusionen beider Zelltypen erreichen. Man dies natürlich auch mittels anderer Technologien erreichen z.B. durch Verwendung von Filtern, mechanischen Vorrichtungen oder auch mittels Ultraschall, um nur einige Beispiele zu nennen.

Alle im folgenden gemachten Spannungsangaben beziehen sich auf einen Elektrodenabstand von 0,2 mm. Dieser Abstand ist nicht zwingen. Es können durchaus auch Kammern oder Strukturen mit anderen Elektrodenabständen eingesetzt werden. Für den Fall müssen lediglich die angegebenen Spannungen entsprechend umgerechnet werden.

In vorteilhaften Ausgestaltungen der Erfindung ist vorgesehen, daß das Pre-Alignment bei einer Wechselspannung zwischen 5 - 20 Volt in einem Zeitraum zwischen 30 Sekunden und 3 Minuten durchgeführt wird. Die Frequenz der Spannung kann zwischen 1 kHz und 4 MHz gewählt werden. Bevorzugt ausgewählte Bedingungen sind 10 V (2 MHz) über einen Zeitraum von 60 Sekunden.

In dem zweiten Schritt wird erfindungsgemäß mindestens ein Puls mit einer Spannung zwischen 15 und 50 Volt Gleichspannung und einer Dauer von 15 µsec bis 1 msec angelegt. Bevorzugt wird ein Puls mit einer Spannung von 20 Volt mit 40 µsec Dauer angewendet. Der Puls ist vorzugsweise ein Rechteckpuls. Denkbar ist aber auch, daß die Spannung während des Pulses ab- oder zunimmt.

Das Post-Alignment wird bei einer Wechselspannung zwischen 2,5 - 10 Volt über einen Zeitraum zwischen 5 Sekunden und 60 Sekunden durchgeführt.. Die Frequenz der Spannung kann zwischen 1 kHz und 4 MHz gewählt werden. Bevorzugt ausgewählte Bedingungen sind 5 V (2 MHz) über einen Zeitraum von 30 Sekunden.

### Im folgenden soll die Erfindung anhand eines Beispiels näher erläutert werden:

Das Beispiel betrifft die Fusion von dendritischen Zellen mit einer permanenten Mama-Tumorzelllinie.

In einem ersten Schritt wurden beide Zellinien geerntet und gezählt. Es wurden dann jeweils 2 x 10⁶ Zellen von jeder Zellinie zusammen pipettiert. Anschließend wurde bei 1200 U/min für 10 min. sedimentiert. Die gemischten Zellen wurden dann zweimal mit Waschmedium (s.u.) gewaschen und jeweils abzentrifugiert.

Das Pellet wurde dann in 220 µml Fusionsmedium (s.u.) aufgenommen und in eine Elektrodenkammer eingefüllt. Nach Überführung in das Elektrofusionsmedium muß die weitere Aufarbeitung sehr zügig erfolgen, da die Zellen hypoosmotische Bedingungen nur über einen kurzen Zeitraum tolerieren. Der Elektrodenabstand betrug 0,2 mm. Die Fusionsbedingungen waren wie folgt:
Das Pre-Alignment wurde für 60 Sekunden bei 10 Volt Wechselspannung (2 MHz) durchgeführt. Es folgte ein Puls von 40 Mikrosekunden Dauer mit 20 Volt Gleichspannung. Das Post-Alignment wurde für 30 Sekunden bei 5 Volt Wechselspannung durchgeführt.
Man ließ dann die Kammer für 10 Minuten bei Raumtemperatur verweilen. Anschließend wurde mit 1 ml Phosphat gepufferter Kochsalzlösung (PBS) gespült und die fusionierten Zellen wurden für 30 Minuten im Brutschrank bei 37°C inkubiert. Denkbar sind auch längere Inkubationszeiten bis zu 12 h.
Danach wurden die Zellen bei 1200 U/min für 10 Minuten abzentrifugiert und auf das gewünschte Volumen eingestellt (vorzugsweise in 5 % Glukoselösung).
Waschmedium und Elektrofusionsmedium wurden unter Verwendung folgender Lösungen hergestellt:

### Lösung 1:

250 ml Sorbitol-Infusionslösung 40
250 ml Ampuwa Fresenius
200 µl Calcium Braun
780 µl Magnesium Verla

### Lösung 2:

500 ml Ampuwa Fresenius
200 µl Calcium Braun
780 µl Magnesium Verla

Zur Herstellung des Elektrofusionsmdiums wurden 27 ml Lösung 1 mit 472,7 ml Lösung 2 vermischt, dem Gemisch 5,5 ml entnommen und mit 5,5 ml Lösung 1 vermischt.

Zur Herstellung des Waschmediums wurden 109 ml Lösung 1 mit 391 ml Lösung 2 vermischt, dem Gemisch 17,5 ml entnommen und mit 17,5 ml Lösung 1 vermischt.

## Patentansprüche

1. Verfahren zur Fusion von dendritischen Zellen mit Tumorzellen, bei dem dendritische Zellen mit Tumorzellen vermischt, ggf. mit einem Waschmediumgewaschen und dann zentrifugiert werden, das Pellet in einem Elektrofusionsmedium aufgenommen und dann mittels Elektrofusion behandelt wird, **dadurch gekennzeichnet, daß** das Elektrofusionsmedium eine Lösung mit folgenden Inhaltsstoffen in pyrogenfreiem, sterilem destilliertem Wasser ist:
20 mM/l - 150 mM/l Zucker,
0,05mM/l - 1mM/l Magnesiumsalz und
0,01mM/l - 1mM/l Calciumsalz.

2. Verfahren nach Anspruch 1, **dadurch gekennzeichnet, daß** die Zuckerkonzentration 75 mM/l, die Magnesiumsalzkonzentration 0,5 mM/l und die Calciumsalzkonzentration 0,1 mM/l ist.

3. Verfahren nach Anspruch 1 oder 2, **dadurch gekennzeichnet, daß** als Zucker Sorbit oder Glukose eingesetzt wird.

4. Verfahren nach einem der vorhergehenden Ansprüche, **dadurch gekennzeichnet, daß** als Zucker anteilig Trehalose eingesetzt wird.

5. Verfahren nach einem der vorhergehenden Ansprüche, **dadurch gekennzeichnet, daß** das Elektrofusionsmedium eine Leitfähigkeit von 70- 90 µS, einen pH-Wert von ca. 6,6 - 7,0 und eine Osmolarität von 70 mOsm aufweist.

6. Verfahren nach einem der vorhergehenden Ansprüche, **dadurch gekennzeichnet, daß** das Waschmedium eine Lösung mit folgenden Inhaltsstoffen in pyrogenfreiem, sterilem destilliertem Wasser ist:
270 mM/l- 310 mM/l Zucker,
0,05mM/l - 1mM/l Magnesiumsalz und
0,01mM/l - 1mM/l Calciumsalz.

7. Verfahren nach Anspruch 6, **dadurch gekennzeichnet, daß** das Waschmedium eine Leitfähigkeit von Leitfähigkeit von ca. 75µS, einem pH-Wert von ca. 6,3 - 7,0 und einer Osmolarität von ca 280 mOsm aufweist.

8. Verfahren nach einem der vorhergehenden Ansprüche, **dadurch gekennzeichnet, daß** im Waschmedium und/oder Elektrofusionsmedium der Zucker anteilig durch Polyethyleglykol ersetzt wird.

9. Verfahren nach einem der vorhergehenden Ansprüche, **dadurch gekennzeichnet, daß** bei der Elektrofusion in einem ersten Schritt (Pre-Alignment) eine Feldstärke zwischen 250 bis 1000 Volt/cm Wechselspannung über einen Zeitraum zwischen 30 Sekunden und 3 Minuten angelegt wird.

10. Verfahren nach einem der vorhergehenden Ansprüche, **dadurch gekennzeichnet, daß** bei der Elektrofusion in einem zweiten Schritt (Puls) mindestens ein Puls mit einer Feldstärke 750 bis 2500 Volt/cm Gleichspannung und einer Dauer von 15 µsec bis 1 msec angelegt wird

11. Verfahren nach einem der vorhergehenden Ansprüche, **dadurch gekennzeichnet, daß** bei der Elektrofusion in einem dritten Schritt (Post-Alignment) eine Feldstärke zwischen 125 - 500 Volt/cm Wechselspannung über einen Zeitraum zwischen 5 Sekunden und 60 Sekunden angelegt wird.

12. Verfahren nach einem der vorhergehenden Ansprüche, **dadurch gekennzeichnet, daß** die Frequenz im ersten und dritten Schritte der Elektrofusion zwischen 1kHz und 4 MHz liegt.

13. Verfahren nach einem der vorhergehenden Ansprüche, **dadurch gekennzeichnet, daß** die Elektrofusion in einer Mikro- oder Nanoelektrodenstrukturen durchgeführt wird, die mittels Halbleitertechnologie hergestellte Elektroden aufweist.

14. Verfahren nach Anspruch 13, **dadurch gekennzeichnet, daß** die Elektrofusion automatisiert wird.

15. Verfahren nach einem der vorhergehenden Ansprüche, **dadurch gekennzeichnet, daß** die Zellen der unterschiedlichen Typen gezielt pärchenweise zusmammengeführt werden.

16. Verfahren nach einem der vorhergehenden Ansprüche, **dadurch gekennzeichnet, daß** man nach dem dritten Schritt die Zellen 10 min bei Raumtemperatur ruhen läßt, danach physiologische Pufferbedingungen herstellt und dann mind. 30 min bei 37°C inkubiert.

## Claims

1. A process for the fusion of dendritic cells with tumor cells in which dendritic cells are combined with tumor cells, if necessary washed using a washing medium and then centrifuged, the pellet suspended in an electrofusion medium and then treated by electrofusion, **characterised in that** the electrofusion medium is a solution containing the following substances of content in pyrogen-free, sterile distilled water:
20 mMl1-150 mM/l sugar,
0.05 mM/l-1 mM/l magnesium salt and
0.01 mM/l-1 mM/l calcium salt.

2. A process according to claim 1, **characterised in that** the sugar concentration is 75 mM/l, the magnesium salt concentration is 0.5 mM/l and the calcium salt concentration is 0.1 mM/l.

3. A process according to claim 1 or 2, **characterised in that** the sugar used is sorbitol or glucose.

4. A process according to one of the preceding claims, **characterised in that** the sugar used is partly trehalose.

5. A process according to one of the preceding claims, **characterised in that** the electrofusion medium has a conductivity of 70-90 µS, a pH value of approximately 6.6-7.0 and an osmolarity of 70 mOsm.

6. A process according to one of the preceding claims, **characterised in that** the washing medium is a solution containing the following substances of content in pyrogen-free, sterile distilled water:
270 mM/l-310 mM/l sugar,
0.05 mM/l-1 mM/l magnesium salt and
0.01 mM/l-1 mM/l calcium salt

7. A process according to claim 6, **characterised in that** the washing medium has a conductivity of 75 µS, a pH value of approximately 6.3-7.0 and an osmolarity of 280 mOsm.

8. A process according to one of the preceding claims, **characterised in that** in the washing medium and/or the electrofusion medium the sugar is partly replaced by polyethylene glycol.

9. A process according to one of the preceding claims, **characterised in that** in a first step (pre-alignment) of the electrofusion process a field intensity of between 250 and 1,000 Volt/cm alternating current voltage is applied over a period of between 30 seconds and 3 minutes.

10. A process according to one of the preceding claims, **characterised in that** in a second step (fusion pulse) of the electrofusion process at least one pulse using a field intensity of 750 to 2,500 Volt/cm direct current voltage is applied over a period of 15 µsec to 1 msec.

11. A process according to one of the preceding claims, **characterised in that** in a third step (post-alignment) of the electrofusion process a field intensity of between 125 and 500 Volt/cm alternating current voltage is applied over a period of between 5 seconds and 60 seconds.

12. A process according to one of the preceding claims, **characterised in that** the frequency in the first and the third steps of the electrofusion process is between 1 kHz and 4 MHz.

13. A process according to one of the preceding claims, **characterised in that** the electrofusion is carried out in a micro- or nanoelectrode structure that has electrodes manufactured using semiconductor technology.

14. A process according to claim 13, **characterised in that** the electrofusion process is automated.

15. A process according to claim 1, **characterised in that** the cells of the different cell types are brought together targetedly paired.

16. A process according to one of the preceding claims, **characterised in that** following the third step one allows the cells to rest for 10 minutes at room temperature, thereafter establishes physiological buffer conditions, and then incubates for at least 30 minutes at 37°C.

## Revendications

1. Procédé de fusion de cellules dendritiques avec des cellules tumorales consistant à mélanger des cellules dendritiques avec des cellules tumorales puis, après les avoir éventuellement lavées à l'aide d'un agent de lavage, à les centrifuger, après quoi le culot est indroduit dans un milieu d'électrofusion puis traité par électrofusion, **caractérisé en ce que** le milieu d'électrofusion est une solution avec les composants suivants contenus dans de l'eau distillée stérile non pyrogène:
20 mM/l- 150 mM/l de sucre,
0,05 mM/l - 1 mM/l de sel de magnésium et
0,01 mM/l - 1 mM/l de sel de calcium.

2. Procédé selon la revendication 1, **caractérisé en ce que** la concentration de sucre est de 75 mM/l, la concentration de sel de magnésium de 0,5 mM/l et la concentration de sel de calcium de 0,1 mM/l.

3. Procédé selon la revendication 1 ou 2, **caractérisé en ce que** l'on emploie du sorbitol ou du glucose en guise de sucre.

4. Procédé selon l'une des revendications précédentes, **caractérisé en ce que** l'en emploie partiellement du tréhalose en guise de sucre.

5. Procédé selon l'une des revendications précédentes, **caractérisé en ce que** le milieu d'électrofusion présente une conductivité de 70 - 90 µS, un pH d'env. 6,6 - 7,0 et une osmolarité de 70 mOsm.

6. Procédé selon l'une des revendications précédentes, **caractérisé en ce que** l'agent de lavage est une solution avec les composants suivants contenus dans de l'eau distillée stérile non pyrogène :
270 mM/l- 310 mM/l de sucre,
0,05 mM/l- 1 mM/l de sel de magnésium et
0,01 mM/l- 1 mM/l de sel de calcium.

7. Procédé selon la revendication 6, **caractérisé en ce que** l'agent de lavage présente une conductivité d'env. 75µS, un pH d'env. 6,3 - 7,0 et une osmolarité d'env. 280 mOsm.

8. Procédé selon l'une des revendications précédentes, **caractérisé en ce que** le sucre dans l'agent de lavage et/ou le milieu d'électrofusion est partiellement remplacé par du polyéthylèneglycol.

9. Procédé selon l'une des revendications précédentes, **caractérisé en ce que**, lors de l'électrofusion, on applique en une première étape (pré-alignement) une intensité de champ comprise entre 250 et 1000 Volt/cm de tension alternative pour une durée comprise entre 30 secondes et 3 minutes.

10. Procédé selon l'une des revendications précédentes, **caractérisé en ce que**, lors de l'électrofusion, on applique en une seconde étape (impulsion) au moins une impulsion d'intensité de champ comprise entre 750 et 2500 Volt/cm de tension continue pour une durée comprise entre 15 µs et 1 ms.

11. Procédé selon l'une des revendications précédentes, **caractérisé en ce que,** lors de l'électrofusion, on applique en une troisième étape (post-alignement) une intensité de champ comprise entre 125 et 500 Volt/cm de tension alternative pour une durée comprise entre 5 secondes et 60 secondes.

12. Procédé selon l'une des revendications précédentes, **caractérisé en ce que** la fréquence au cours de la première et de la troisième étape de l'électrofusion est comprise entre 1 kHz et 4 MHz.

13. Procédé selon l'une des revendications précédentes, **caractérisé en ce que** l'électrofusion est réalisée dans une structure à microélectrodes ou nanoélectrodes dotée d'électrodes réalisées au moyen d'une technologie des semi-conducteurs.

14. Procédé selon la revendication 13, **caractérisé en ce que** l'électrofusion est automatisée.

15. Procédé selon l'une des revendications précédentes, **caractérisé en ce que** les cellules des différents types sont fusionnées par paires de façon ciblée.

16. Procédé selon l'une des revendications précédentes, **caractérisé en ce que**, après la troisième étape, on laisse les cellules reposer à température ambiante pendant 10 min., après quoi on crée des conditions de milieu physiologique tampon dans lequel laisse incuber pendant au moins 30 min. à 37°C.
